# EUROPEAN PATENT SPECIFICATION

(11) **EP 0 685 214 B1**
(45) Date of publication and mention of the grant of the patent: **13.02.2002**
(21) Application number: 95303755.3
(22) Date of filing: 01.06.1995
(51) Int. Cl.: A61F 13/15, A61F 13/46, A61F 13/50

(54) **Disposable body fluids absorbent padding**
Wegwerfbares Kissen zum Absorbieren von Körperflüssigkeiten
Coussin à jeter pour absorber des liquides corporels

(30) Priority: 03.06.1994 JP 12258494
(43) Date of publication of application: 06.12.1995
(73) Proprietor: UNI-CHARM CORPORATION, Kawanoe-shi Ehime-ken (JP)
(72) Inventor: Mizutani, Satoshi, Kawanoe-shi, Ehime-ken (JP)
(74) Representative: Murgatroyd, Susan Elizabeth

(56) References cited:
- EP-A- 0 059 498
- WO-A-88/05269
- GB-A- 2 114 445
- US-A- 2 788 003
- US-A- 4 590 114
- US-A- 4 798 603

## Description

The present invention relates to a disposable body fluids absorbent padding and, more particulary, to a body fluids padding such as menstruation pads, diapers for infants, diapers for incontinence and the like.

It is well known in making such paddings that a topsheet is generally made from hydrophobic materials, of which the inner surface is covered with a liquid-guiding fibrous layer being less hydrophobic than the topsheet or rather hydrophilic in contact with a liquid-absorbent core in order to alleviate a feeling of wetness due to the body fluids during use of the padding by rapidly guiding the body fluids discharged over the tophseet into the core. For example, Japanese Laid-Open Patent Application No. 1982-1340 discloses a technique according to which a topsheet made of a hydrophobic perforated plastic film is applied on its lower surface by means of suitable adhesive, then fibers being less hydrophobic than the topsheet are flocked on the adhesive to form a thin layer and this thin layer is put against a liquid-absorbent core so that the body fluids may be more rapidly transferred to the core than in the case of the topsheet without the thin layer.

While the technique disclosed in the above-identified literature tends to bond the thin layer extending over the lower surface of the topsheet integrally to the topsheet by means of adhesive applied to the lower surface, the thin layer may have some portions which can hardly or can never come in contact with adhesive, depending on a thickness of the thin layer and such portions would be hardly bonded to the topsheet or even spaced from the topsheet. Should the body fluids absorbent padding be bent during its actual use, component fibers of the thin layer will be loosened from one another or peeled off from the topsheet and passages of the body fluids established by capillary action will be interrupted there. Consequently, the body fluids will be prevented from being rapidly transferred to the core and stay on the upper surface of the topsheet for a long time, causing stuffiness or eruption. In addition, such interruption of the passages will be apt to cause the body fluids to leak sideways.

GB-A-2114445 discloses a disposable body fluid absorbent padding having a liquid-permeable topsheet, a liquid-impermeable backsheet and a liquid-absorbent core disposed therebetween. A liquid-guiding fibrous layer is interposed between the topsheet and the core in order to guide body fluids from the topsheet to the core. The fibrous layer and the topsheet both contain hot melt materials, the layers being integrally bonded together at a plurality of intermittent spots in the direction of a thickness defined between the upper surface of the top sheet and the lower surface of the liquid-guiding fibrous layer.

It is an object of the invention to provide a disposable body fluids absorbent padding which enables rapid transfer of body fluids to the core as well as reducing the possibility of leakage of body fluids from the sides of the padding.

Accordingly, the present invention consists in a disposable body fluids absorbent padding comprising a liquid-permeable topsheet, a liquid-impermeable backsheet, a liquid-absorbent core disposed between these two sheets and a liquid-guiding fibrous layer being more hydrophilic than said topsheet and interposed between said topsheet and said liquid-absorbent core in order to guide the body fluids from said topsheet into said core, hot melt materials contained in said topsheet and said liquid-guiding fibrous layer, respectively, being compressed and fused at a plurality of spots arranged intermittently in a plane of said topsheet whereby said topsheet and said liquid-guiding fibrous layer are integrally bonded together at said spots in the direction of a thickness of said padding, said fused spots extending between the upper surface of said topsheet and the lower surface of said liquid-guiding fibrous layer, characterised in that:
said plurality of fused spots are arranged in intermittent compressed lines forming grooves extending diagonally with respect to a longitudinal axis of said padding to form a lattice pattern; and
the peripheries of said topsheet and said backsheet extend outwardly beyond the periphery of said core, said topsheet and said backsheet being bonded together along their peripheries by a seal line which forms a groove extending around said core.

The disposable body fluids absorbent padding constructed as described above is advantageous in that, even during its actual use, the component fibers of
the liquid-guiding fibrous layer are neither loosened from one another nor peeled off from the lower surface of the topsheet at and adjacent the fused spots independently of its thickness, since the top-sheet and the liquid-guiding fibrous layer are integrally bonded to each other in the direction of thickness by fusion of the hot melt material contained in them, respectively.

The invention will now be described in more detail, by way of example, with reference to the accompanying drawings, wherein:-
Fig. 1 is a perspective view showing an embodiment of the invention in the form of a menstruation pad as partially broken away; and
Fig. 2 is a sectional view taken along a line X - X in Fig. 1.

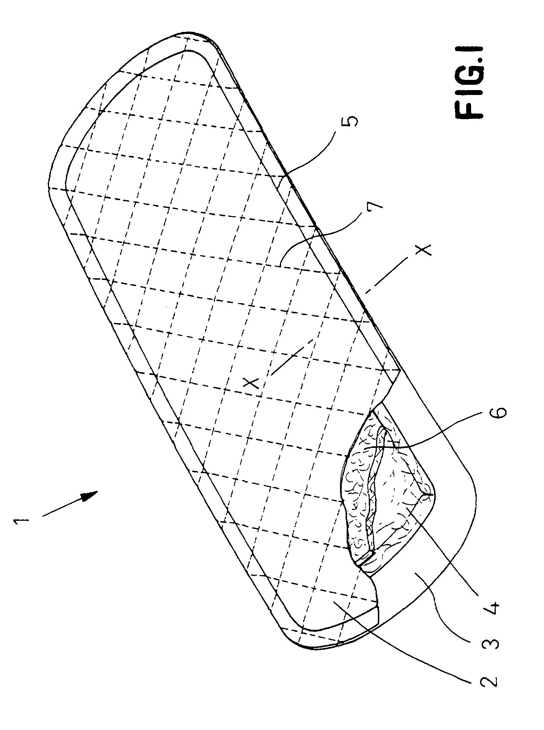

Referring to Fig. 1, a menstruation pad 1 comprises a liquid-permeable topsheet 2, a liquid-impermeable backsheet 3 and a liquid-absorbent core 4 disposed between these two sheets 2, 3 wherein the top- and backsheets 2, 3 are water-tightly bonded together by a seal line 5 along their portions extending outward beyond a peripheral edge of the core 4. As will be described later in more detail, a liquid-guiding fibrous layer 6 is bonded to the lower (i.e., not skin-contacting) surface of the topsheet 2 and put against the core 4. Over the upper surface of the topsheet 2, a plurality of fused spots are arranged intermittently as individually viewed but in a lattice pattern when they are viewed as a whole. The topsheet 2 is preferably made of a hydrophobic sheet containing therein hot melt materials, specifically a nonwoven fabric containing 5% or higher by weight of thermoplastic synthetic fibers, split yarns fibrillated from a thermoplastic synthetic resin film or a mixture thereof or a liquid-permeable perforated thermoplastic synthetic resin film. The fibrous layer 6 comprises suitable fibrous materials such as a nonwoven fabric containing 5% or higher by weight of hot melt fibrous materials inclusive of said split yarns and less hydrophobic than the topsheet 2. The fibrous layer 6 is also lower than the topsheet 2 in a fibrous density and higher in a cushion. While the fibrous materials similar to those of the topsheet 2 may be used to form the fibrous layer 6, such fibrous materials are preferably subjected to an appropriate treatment so as to be less hydrophobic than the topsheet, i.e., so as to become rather hydrophilic.

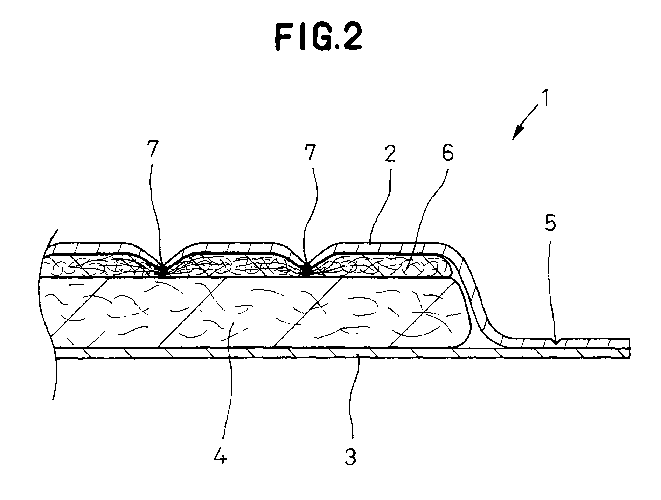

Referring to Fig. 2, the topsheet 2 and the fibrous layer 6 are integrally bonded together in the direction of thickness defined between the upper surface of the topsheet 2 and the lower surface of the fibrous sheet 6 at fused spots 7 by fusion of the hot materials contained in them, respectively. In consequence, the component fibers of these topsheet 2 and fibrous layer 6 are neither loosened nor peeled off from one another at and adjacent the fused spots 7, independently of their thicknesses. A distance between each pair of adjacent fused spots 7 may be appropriately selected not only to maintain the topsheet 2 and the fibrous layer 6 in close contact with each other but also to maintain their component fibers reliably intertwined, respectively. Each of the fused spots 7 is compressed to have a high density and to form a groove along which the body fluids may diffusively flow and rapidly guide them into the core 4. In this manner, the core 4 is optimally utilized.

In the pad 1, menstrual blood discharged onto the upper surface of the topsheet 2 is guided to the relatively hydrophilic fibrous layer 6 immediately underlying the topsheet 2 and then transferred into the core 4. The fibrous layer 6 functions to transfer the menstrual blood into the core 4 under the capillary action provided by its component fibers and the fused spots 7 serve to assure that said capillary action is not interrupted in the direction of thickness by maintaining the upper surface of the fibrous layer 6 in close contact with the lower surface of the topsheet 2 and at the same time by maintaining the component fibers of the fibrous layer 6 reliably intertwined between the upper and lower surfaces of the fibrous layer 6.

The hot melt materials as the components of the topsheet 2 and the fibrous layer 6 may be a film or fibers, for example, of polyethylene, polypropylene, nylon, polyester resin or composite fibers thereof. Alternatively, hydrophilic materials such as rayon fibers or pulp fibers up to 20% by weight may be mixed into the above-mentioned materials. The backsheet 3 and the liquid-absorbent core 4 may be of the materials usually used as the materials for these members. To fuse the topsheet 2 and the fibrous layer 6 together, the conventional technique such as heat embossing may be used in a pattern of spots as presented by the fused spots 7 in Figs. 1 and 2, or straight lines, curved lines or combination of these lines and spots.

The disposable body fluids absorbent padding according to the invention is advantageous in that the topsheet and the liquid-guiding fibrous layer are never peeled off from each other and the component fibers contained in the sheet and layer are never loosened from one another independently of their thicknesses, since the fibrous layer interposed between the topsheet and the liquid-absorbent core is integrally bonded to the topsheet in the direction of their thicknesses.

Accordingly, even if the padding is bent or wrinkled during its use, the body fluids can be rapidly transferred from the upper surface of the topsheet into the liquid-absorbent core without occurrence of leaking sideways.

## Claims

1. A disposable body fluids absorbent padding comprising a liquid-permeable topsheet (2), a liquid-impermeable backsheet (3), a liquid-absorbent core (4) disposed between these two sheets (2,3) and a liquid-guiding fibrous layer (6) being more hydrophilic than said topsheet (2) and interposed between said topsheet (2) and said liquid-absorbent core (4) in order to guide the body fluids from said topsheet (2) into said core (4), hot melt materials contained in said topsheet (2) and said liquid-guiding fibrous layer (6), respectively, being compressed and fused at a plurality of spots (7) arranged intermittently in a plane of said topsheet (2) whereby said topsheet (2) and said liquid-guiding fibrous layer (6) are integrally bonded together at said spots (7) in the direction of a thickness of said padding, said fused spots (7) extending between the upper surface of said topsheet (2) and the lower surface of said liquid-guiding fibrous layer (6), **characterised in that**:
said plurality of fused spots (7) are arranged in intermittent compressed lines forming grooves extending diagonally with respect to a longitudinal axis of said padding to form a lattice pattern; and
the peripheries of said topsheet and said backsheet extend outwardly beyond the periphery of said core, said topsheet and said backsheet being bonded together along their peripheries by a seal line (5) which forms a groove extending around said core (4).

2. A disposable body fluids absorbent padding according to claim 1, wherein said lattice pattern of grooves formed by said fused spots (7) extends outwardly beyond the periphery of said core (4).

3. A disposable body fluids absorbent padding according to claim 2, wherein the seal line groove (5) intercepts the lattice pattern grooves.

4. A disposable body fluids absorbent padding according to claim 1, 2 or 3, wherein each of said fused spots (7) is compressed to have a high density.

## Patentansprüche

1. Wegwerfbares Kissen zum Absorbieren von Körperflüssigkeiten, das eine flüssigkeitsdurchlässige Oberschicht (2), eine flüssigkeitsundurchlässige Unterschicht (3), einen Flüssigkeit absorbierenden Kern (4), derzwischen diesen beiden Schichten (2, 3) angeordnet ist, und eine Flüssigkeit leitende Faserschicht (6), die stärker wasseraufsaugend als die Oberschicht (2) ist und die zwischen der Oberschicht (2) und dem Flüssigkeit absorbierenden Kern (4) angeordnet ist, um die Körperflüssigkeiten von der Oberschicht (2) in den Kern (4) zu leiten, Heißschmelzmaterialien, die jeweils in der Oberschicht (2) und der Flüssigkeit leitenden Faserschicht (6) enthalten sind, die an einer Vielzahl von Punkten (7), die mit Unterbrechungen in einer Ebene der Oberschicht (2) angeordnet sind, verdichtet und geschmolzen sind, umfaßt, wobei die Oberschicht (2) und die Flüssigkeit leitende Faserschicht (6) an allen Punkten (7) in der Richtung einer Dicke des Kissens angeschweißt sind, wobei sich die geschmolzenen Punkte (7) zwischen der oberen Fläche der Oberschicht (2) und der unteren Fläche der Flüssigkeit leitenden Faserschicht (6) erstrecken, **dadurch gekennzeichnet, dass**:
die Vielzahl geschmolzener Punkte (7) mit Unterbrechungen in verdichteten Linien angeordnet sind, die Nuten bilden, die sich diagonal zu einer Längsachse des Kissens erstrecken und so ein Gittermuster bilden; und dass
sich die Umfangsbegrenzung der Oberschicht und diejenige der Unterschicht nach außen hin über die Umfangsbegrenzung des Kerns hinaus erstrecken, wobei die Oberschicht und die Unterschicht durch eine Versiegelungslinie (5), die eine Nut bildet, die sich um den Kern (4) herum erstreckt, entlang ihrer Umfangsbegrenzung miteinander verbunden sind.

2. Wegwerfbares Kissen zum Absorbieren von Körperflüssigkeiten nach Anspruch 1, wobei das Gittermuster der durch die geschmolzenen Punkte (7) gebildeten Nuten sich nach außen hin über die Umfangsbegrenzung des Kerns (4) hinaus erstreckt.

3. Wegwerfbares Kissen zum Absorbieren von Körperflüssigkeiten nach Anspruch 2, wobei die Versiegelungsliniennut (5) die Gittermusternuten schneidet.

4. Wegwerfbares Kissen zum Absorbieren von Körperflüssigkeiten nach Anspruch 1, 2 oder 3, wobei jeder der geschmolzenen Punkte (7) zusammengedrückt ist, um so eine hohe Dichte zu haben.

## Revendications

1. Elément absorbant jetable pour absorber les fluides corporels comprenant une feuille supérieure perméable au liquide (2), une feuille arrière imperméable au liquide (3), un noyau absorbant le liquide (4) disposé entre ces deux feuilles (2, 3) et une couche fibreuse de guidage de liquide (6) qui est plus hydrophile que ladite feuille supérieure (2) et qui est interposée entre ladite feuille supérieure (2) et ledit noyau absorbant le liquide (4) afin de guider les fluides corporels à partir de la feuille supérieure (2) dans ledit noyau (4), des matériaux thermofusibles contenus dans ladite feuille supérieure (2) et ladite couche fibreuse de guidage de liquide (6), respectivement, étant comprimés et fusionnés en une pluralité de points (7) arrangés de manière intermittente dans un plan de ladite feuille supérieure (2) de sorte que ladite feuille supérieure (2) et ladite couche fibreuse de guidage de liquide (6) sont liées ensemble de manière monobloc au niveau desdits points (7) dans la direction d'une épaisseur dudit élément absorbant, lesdits points fusionnés (7) s'étendant entre la surface supérieure de ladite feuille supérieure (2) et la surface inférieure de ladite couche fibreuse de guidage de liquide (6), **caractérisé en ce que** :
ladite pluralité de points fusionnés (7) sont arrangés en lignes comprimées de manière intermittente formant des rainures s'étendant diagonalement par rapport à un axe longitudinal dudit élément absorbant pour former un motif de réseau ; et
les périphéries de ladite feuille supérieure et de ladite feuille arrière s'étendent vers l'extérieur au-delà de la périphérie dudit noyau, ladite feuille supérieure et ladite feuille arrière étant liées ensemble le long de leur périphérie par une ligne de soudage (5) qui forme une rainure s'étendant autour dudit noyau (4).

2. Elément absorbant jetable pour absorber les fluides corporels selon la revendication 1, dans lequel ledit motif de réseau de rainures formé par lesdits points fusionnés (7) s'étend vers l'extérieur au-delà de la périphérie dudit noyau (4).

3. Elément absorbant jetable pour absorber les fluides corporels selon la revendication 2, dans lequel la rainure de ligne de soudage (5) croise les rainures du motif de réseau.

4. Elément absorbant jetable pour absorber les fluides corporels selon la revendication 1, 2 ou 3, dans lequel chacun des points fusionnés (7) est comprimé pour avoir une haute densité.
